# EUROPÄISCHE PATENTANMELDUNG

(11) **EP 2 392 281 A1**
(43) Veröffentlichungstag der Anmeldung: **07.12.2011**
(21) Anmeldenummer: 10168268.0
(22) Anmeldetag: 02.07.2010
(51) Int. Cl.: A61B 18/14

(54) **Elektrochirurgisches bipolares Skalpell**

(30) Priorität: 20.04.2010 LV 100058
(71) Anmelder: OOO "Novye Energeticheskie, Moskau 107045 (RU); "Golsen Limited", 3041 Limassol (CY)
(72) Erfinder: Belov, Sergey V., 119311 Moskow (RU); Ganin, Ygor Y., 124498 Moskow (RU); Borik, Mikhail A., 105264 Moskow (RU); Danileyko, Yuri K., 142092 Troitsk (RU); Ivanov, Alexander D., 109378 Moskow (RU); Lomonova, Yelena J., 129090 Moskow (RU); Nefedov, Sergey M., 115612 Moskow (RU); Osiko, Vjacheslav V., 117420 Moskow (RU); Salyuk, Viktor A., 117321 Moskow (RU)
(74) Vertreter: Jeck, Anton

(57) **Zusammenfassung**

Die Erfindung betrifft ein elektrochirurgisches Skalpell, das in der medizinischen Technik und zwar als medizinisches elektrochirurgisches Instrument einsetzbar ist. Es kann bei der Durchführung von chirurgischen Eingriffen zur Durchtrennung und gleichzeitigen Koagulation von biologischem Gewebe und Blutgefäße eingesetzt werden. Ein kristallines nanostrukturiertes teilweise stabilisiertes Zirkondioxid ist in einer Verbundstoffstruktur der Klinge als Dielektrikum benutzt. Das Zirkondioxid ist biologisch mit Geweben eines Lebewesens verträglich, weist eine absolute chemische Inaktivität, einen breiten Arbeitstemperaturbereich und eine erhöhte Verschleißfestigkeit auf und stellt während der Durchtrennung eine Koagulation des Biogewebes sicher.

## Beschreibung

Die Erfindung betrifft ein elektrochirurgisches bipolares Skalpell nach dem Oberbegriff des Anspruchs 1.

Die Erfindung ist als medizinisches elektrochirurgisches Instrument einsetzbar und kann bei der Durchführung von chirurgischen Eingriffen für die Durchtrennung und gleichzeitige Koagulation der biologischen Gewebe und Blutgefäße angewendet werden.

Aus der US 4651734 ist ein elektrochirurgisches bipolares Instrument bekannt. Das bekannte elektrochirurgische Instrument besteht aus einer Klinge, einer zusätzlichen bipolaren Elektrode und einem isolierten Griff mit Kontakten für eine Stromeinspeisung. Dabei ist die Klinge aus einem medizinischen Stahl gefertigt. Der Mangel dieses bipolaren Instrumentes ist seine Mitwirkung beim Koagulationsvorgang der Metallelektrode. Ein Teil dieser Elektrode bildet gleichzeitig die Schneideklinge. Trotz der speziellen Behandlung und Härtung der Metallklinge, die unmittelbar das Biogewebe berührt, kommt ihre schnelle Degradation beim Gebrauch infolge der elektrochemischen Abläufe zustande. Dabei liegt eine ungewünschte Verletzung der durchgetrennten Biogewebe vor.

Der nächst kommende Stand der Technik gegenüber der vorgeschlagenen Erfindung ist ein elektrochirurgisches Skalpell (RU 2154435). Es enthält einen Griff mit einer daran befestigten Klinge. Der Klingenfuss (Klingengrund) ist aus hochfestem und hochwärmeleitfähigem Saphir gefertigt. Der Klingenfuss ist so angeschliffen, dass eine Schneidekante gebildet wird. An der Seite der Klinge ist ein Mehrzonenheizelement entlang der Schneidekante angeordnet. Die jeweiligen Zonen des Elementes sind an die Ausgänge eines Mehrfachtemperaturreglers angeschlossen. An der Seite der Klinge ist auch ein Mehrzonen-Mikrosensor für Temperatur angeordnet. Die jeweiligen Zonen des Temperaturmikrosensors sind über einen Mehrfach-Temperaturmesswandler angeschlossen.

Die Mängel dieser Einrichtung sind wie folgt:
1) Die Koagulation erfolgt nur dank dem Kontakt zwischen dem Gewebe und der Saphir-Klinge, die durch den erhitzten und neben der Schneidekante dieser Klinge angeordneten Mehrzonen-Heizkörper erwärmt wird. Das verlangsamt wesentlich die Wärmeübertragung in die Tiefe des Gewebes wegen einer zu niedrigen Wärmeleitfähigkeit des Gewebes
2) die hohe Wärmeträgheit der Temperaturschwankung der Klinge.

Es ist Aufgabe der Erfindung, ein elektrochirurgische Skalpell zu entwickeln, welches eine gleichzeitige Koagulation der an die Klinge des elektrochirurgischen Skalpells anliegenden Biogewebe während der Durchtrennung sicherstellt und welches eine erhöhte Verschleißfestigkeit aufweist.

Die gestellte Aufgabe wird durch die Merkmale des Anspruchs 1 gelöst.

Das elektrochirurgische bipolare Skalpell besteht aus einer keramischen Klinge mit isolierten stromleitenden Metallschichten, die in gegenüberliegenden Ebenen angeordnet sind. Auf dem proximalen Ende des Griffes des elektrochirurgischen bipolaren Skalpells sind Kontakte angeordnet, die zum Anlegen eines elektrischen HF-Stroms vorgesehen sind. An dem distalen Ende des Skalpells ist eine Schneideklinge mit einer Verbundstoffstruktur aus Metall, Keramik und Metall angeordnet.

Eine zusätzliche Besonderheit besteht darin, dass in der Verbundstoffstruktur der Klinge ein kristallines nanostrukturiertes teilweise stabilisiertes Zirkondioxid als Keramik-Komponente benutzt ist. Dabei beträgt die Metallstärke in der genannten Verbundstoffstruktur 0,05 bis 0,2 mm. Die Höhe der Schneidekante des Nichtmetall-Teils der keramischen Klinge aus dem kristallinen nanostrukturierten teilweise stabilisierten Zirkondioxid beträgt 0,3 bis 0,8 mm.

Die Erfindung wird anhand eines in den Zeichnungen dargestellten Ausführungsbeispiels näher erläutert. Es zeigen:

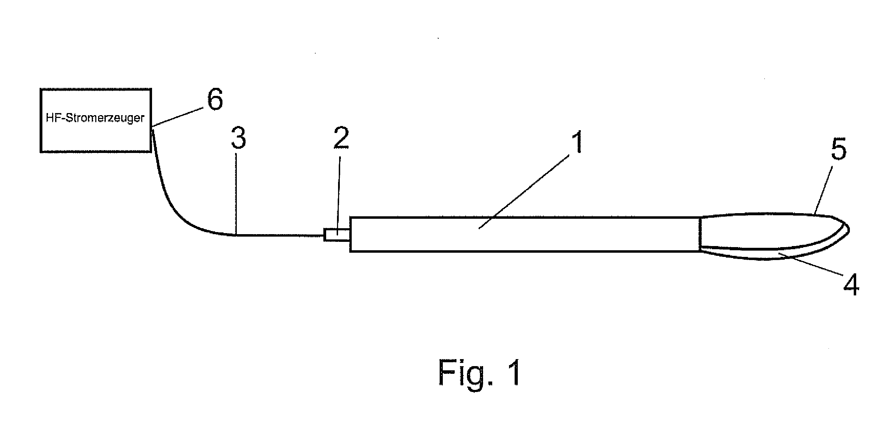

- Fig. 1: ein bipolares elektrochirurgisches Skalpell und

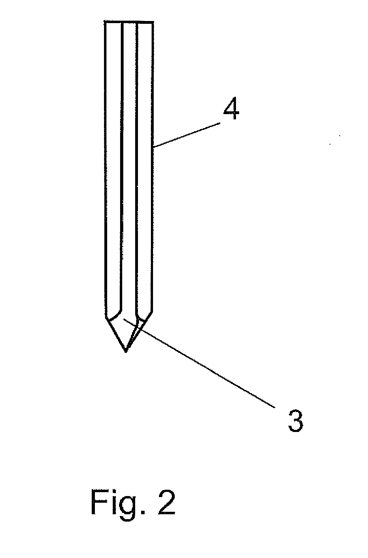

- Fig. 2: eine Klinge des bipolaren elektrochirurgischen Skalpells im Schnitt.

Das elektrochirurgische bipolare Skalpell weist einen isolierten Griff 1 mit Kontakten 2 für die Zuführung eines elektrischen HF-Stroms von einem HF-Stromerzeuger 6, ein keramisches Schneideelement mit einer Schneidekante 4 und zwei Metallaufdampfschichten 5 auf, die als Elektroden dienen. Die Elektroden des bipolaren Skalpells sind mit dem HF-Stromerzeuger mittels eines dünnen Radiofrequenzkabels für die Zuführung des HF-Stroms 3 verbunden.

Die Funktionsweise des vorgeschlagenen bipolaren elektrochirurgischen Skalpells ist wie folgt:

Der HF-Strom wird von dem HF-Stromerzeuger 6 über ein Kabel 3 für die Zuführung des HF-Stroms an die HF-Strom-Kontakte 2 angelegt. Die Kontakte 2 befinden sich an dem proximalen Ende des Griffes 1. Diese Kontakte 2 sind unabhängig voneinander mit Metallelektroden 5 verbunden. Die Metallelektroden 5 sind auf beiden Seiten der keramischen Klinge mit einer Metallaufdampfschicht 5 versehen. Die keramische Klinge ist am distalen Ende des genannten Skalpells angeordnet und weist eine Schneidekante 4 mit einer Verbundstoffstruktur aus Metall, Keramik und Metall auf. Dabei sind die voneinander isolierten leitfähigen Schichten 5 an der Schneideklinge des Instrumentes angeordnet und wirken als aktive bipolare Elektroden, die zwei Berührungspunkte mit dem Biogewebe aufweisen.

Der am Instrument 1 angelegte HF-Strom fließt zwischen den Kontaktpunkten der Elektroden in das Biogewebe und zwar über den kürzesten Weg nur in jenem Bereich des Gewebes, welcher zwischen diesen Punkten liegt. Damit ist die Koagulation hervorgerufen. Wenn der Chirurg den Schnitt macht, wird das biologische Gewebe durch die keramische Klinge des Skalpells mechanisch durchgetrennt. Dabei stellen die auf der Klinge aufgedampften Metallflächen eine Elektrokoagulation sicher, da sie als eine bipolare Elektrode wirken. Bei der Durchtrennung des Biogewebes fängt die Koagulation an, sobald die Metallelektroden der keramischen Klinge das Gewebe berühren. Deswegen legt die Höhe der Schneidekante die Schnitttiefe bis zur Auslösung des Koagulationsvorgangs fest. Da die einstufige Koagulation des Blutgefäßes mit einem Durchmesser von über 1,0 in der HF-Elektrochirurgie problematisch ist, darf die Höhe der Schneidekante den durchschnittlichen Durchmesser von einem "Speiseblutgefäß" nicht überschreiten. Deswegen beträgt die Höhe der Schneidekante bei dem Nichtmetall-Teil der keramischen Klinge aus kristallinem nanostrukturiertem teilweise stabilisiertem Zirkondioxid 0,3 bis 0,8 mm.

In der erwähnten Verbundstoffstruktur der Klinge wird das kristalline nanostrukturierte teilweise stabilisierte Zirkondioxid als Keramik benutzt. Der leitfähige Elektrodenteil der genannten Verbundstoffstruktur ist hergestellt, indem das Metall auf den dielektrischen (keramischen) Teil aufgetragen (abgeschieden) ist. Dabei stellt die Beschichtungsstärke im Bereich von 0,05 - 0,2 mm einerseits eine unwesentliche Stärkenerhöhung der Verbundstoffstruktur der Klinge dar (diese Zunahme beeinflusst nicht die Funktionskennwerte). Andererseits ist eine maximale Haltbarkeit der Abnutzungsfestigkeit gemäß GOST 9.303-84 erreicht.

### Literaturhinweis:

GOST 9.303-84 - Einheitliches System zum Schutz gegen Korosion und Alterung, Metall- und anorganische Nichtmetallbeschichtungen. Allgemeine Auswahlanforderungen

## Patentansprüche

1. Elektrochirurgisches bipolares Skalpell aus einer keramischen Klinge mit isolierten stromleitenden Metallschichten (5), die in gegenüberliegenden Ebenen angeordnet sind,
**dadurch gekennzeichnet,**
**dass** an dem proximalen Ende des Skalpellgriffes (1) Kontakte für eine Zuführung eines elektrischen HF-Stroms (3) und an dem distalen Ende eine Schneideklinge mit einer Verbundstoffstruktur aus Metall, Keramik und Metall angeordnet sind.

2. Skalpell nach Anspruch 1,
**dadurch gekennzeichnet,**
**dass** in der Verbundstoffstruktur der Klinge ein kristallines nanostrukturiertes teilweise stabilisiertes Zirkondioxid als Keramik eingesetzt ist.

3. Skalpell nach Anspruch 2,
**dadurch gekennzeichnet,**
**dass** die Stärke der Metallschichten (5) in der genannten Verbundstoffstruktur im Bereich von 0,05 - 0,2 mm liegt.

4. Skalpell nach Anspruch 2,
**dadurch gekennzeichnet,**
**dass** die Schneidekante am Nichtmetall-Teil der keramischen Klinge aus einem kristallinen nanostrukturierten teilweise stabilisierten Zirkondioxid eine Höhe von 0,3 bis 0,8 mm aufweist.
